Europäisches Patentamt

European Patent Office

Office européen des brevets

(11) Publication number: **0 525 251 A1**

## EUROPEAN PATENT APPLICATION

(21) Application number: **91121886.5**

(22) Date of filing: **20.12.91**

(51) Int. Cl.5: **A61L 15/58**, C09J 153/00

(30) Priority: **31.07.91 US 739202**

(43) Date of publication of application:
**03.02.93 Bulletin 93/05**

(84) Designated Contracting States:
**DE ES FR GB IT**

(71) Applicant: **FINDLEY ADHESIVES INC.**
**11320 Watertown Plank Road**
**Wauwatosa Wisconsin 53226-3413(US)**

(72) Inventor: **Nelson, Kenneth A.**
**N54 W15485 Northway Drive**
**Menomonee Falls, WI 53051(US)**
Inventor: **Alper, Mark D.**
**3245 South Pinewood Creek Court, Apt. No. 105**
**New Berlin, WI 53151(US)**

(74) Representative: **von Füner, Alexander, Dr. et al**
**Patentanwälte v. Füner, Ebbinghaus, Finck**
**Mariahilfplatz 2 & 3**
**W-8000 München 90(DE)**

(54) **Positioning adhesives for absorbent articles.**

(57) An improved positioning adhesive for an absorbent article is a hot melt adhesive blend including a styrene-isoprene-styrene block copolymer (SIS) containing at least 25 parts styrene per 100 parts block copolymer, a compatible tackifying resin and a plasticizing oil.

EP 0 525 251 A1

This application is a continuation in part of Application Serial No. 07/518,056 now abandoned.

## Field of the Invention

The present invention relates to improved hot melt positioning adhesives and absorbent articles employing same, and more particularly to an adhesive which permits an absorbent article to be releasably affixed in a predetermined position to an associated garment for purposes of absorbing all manner of fluids coming into contact with the absorbent article, but which further permits the removal of the absorbent article from the garment without depositing any substantial amounts of adhesive residue on the garment or otherwise disturbing or damaging the fiber composition of the garment.

## Background of the Invention

The prior art is replete with numerous examples of absorbent articles which are manufactured for use in the absorption of menstrual flow, urine, fecal matter and other body fluids. For example, sanitary napkins, panty shields and diaper inserts are provided with pressure sensitive adhesives which are used as positioning adhesives for the purpose of attaching the respective articles to a supporting undergarment and hold it in place during use without transferring to or otherwise being deposited on the undergarment. In addition, the adhesive must not otherwise discolor, damage or disturb the fibers of the garment.

In addition to the aforementioned characteristics, the pressure sensitive adhesives employed for the previously mentioned end-use applications must have an application viscosity that permits the adhesive to readily flow onto and partially penetrate the particular surface to which it is applied, but which further permits a sufficient amount of the adhesive to remain on the exposed surface so that it may releasably secure this same surface to the undergarment. Moreover, the adhesive coating for these same applications must have good bond strength and high tack so that it may assist in the initial placement of the absorbent article on the garment but not transfer to the undergarment or damage it when the absorbent article is removed.

As discussed above, hot melt pressure sensitive adhesives have been employed in the past as adhesives for various features of absorbent articles. More particularly, hot melt pressure sensitive adhesives are disclosed in U.S. Patent Nos. 4,136,699; 4,719,261; 4,411,954; and 4,835,200. Further, Patent No. 4,136,699 and 4,704,110 disclose hot melt adhesives having a composition based upon an A-B-A block copolymer structure. This particular A-B-A structure includes polystyrene end blocks and a rubbery polyolefin midblock. Moreover, the polyolefin midblock may include a poly (ethylenebutylene) block or a teleblock copolymer including at least three branches radially branching out from a central hub with polystyrene terminal blocks and a butadiene segment in the center. In order to achieve the desired properties, however, the composition must contain relatively large amounts of oil. In addition to the foregoing, one of the patents noted above discloses an adhesive containing a styrene-butadiene-styrene triblock (S-B-S) radial or multiblock copolymer which contains about 38 to 55 parts styrene per 100 parts copolymer.

While the above described positioning adhesives containing the S-B-S copolymers operate in an acceptable fashion they do present problems and other difficulties which have detracted from their usefulness. For example, the adhesives noted above, and including the S-B-S copolymers, have been shown to be undesirable from a manufacturing perspective inasmuch as it has been demonstrated that they can, on occasion, gel thereby clogging various pieces of production equipment if operating temperatures and other manufacturing parameters, such as time are exceeded. Further, it has generally been accepted that the manufacturing process for various products would be greatly facilitated if a hot melt pressure sensitive adhesive could be located which would have an acceptable viscosity at the predetermined operating temperatures found during the manufacturing process. This would also, of course, eliminate the earlier described problems which relate to the clogging of manufacturing equipment. A pressure sensitive adhesive having these characteristics would, of course, increase the speed and thus the profitability for manufacturing an absorbent article employing this same hot melt pressure sensitive adhesive.

## Objects and Summary of the Invention

It is therefore an object of the present invention to provide an improved hot melt pressure sensitive adhesive, hereinafter referred to as a positioning adhesive, for use with an absorbent article.

It is a further object of the present invention to provide a positioning adhesive which, when applied to an absorbent article, is adapted to releasably attach the absorbent article to a fibrous substrate, such as a garment, without depositing a substantial amount of residue attributable to the positioning adhesive on the fibrous substrate, and which further does not damage, discolor or otherwise harm the fibrous substrate.

Another object of the present invention is to provide a positioning adhesive which includes about 15 to about 50% by weight of a styrene-isoprene-styrene (SIS) block copolymer containing from about 25 to about 50 parts styrene per 100 parts of copolymer, and which, when combined with about 30% to about 70% by weight of a compatible tackifying resin, and about 5% to about 35% by weight of a suitable plasticizing oil has a viscosity of about 2000 to about 5000 centipoise at a pour temperature of approximately 325°F, and an initial cotton peel adhesion of about 200 to about 400 grams.

Another object of the present invention is to provide a positioning adhesive which may further include 5% or less, by weight, of a suitable wax and about 0.1% to about 2% by weight of an antioxidant and/or stabilizer.

Another object of the present invention is to provide a positioning adhesive which resists gelling and clogging of production machinery under normal manufacturing conditions.

Another object of the present invention is to provide a positioning adhesive which may be applied to the garment facing surface of an absorbent article by numerous methods of application including extrusion, spray, and wheel application methods.

A further object of the present invention is to provide a positioning adhesive wherein the compatible tackifying resin is selected from rosins and modified rosins including hydrogenated versions thereof, glycerol and pentaerythritol esters of rosins and modified rosins including hydrogenated, polyterpene resins, copolymers and terpolymers of natural terpenes, phenolic modified terpenes, aliphatic petroleum hydrocarbon resins including hydrogenated versions, aromatic petroleum hydrocarbon resins including hydrogenated versions and aliphatic/aromatic hydrocarbon resins including hydrogenated versions thereof.

Description of the Preferred Embodiments

In the preferred practice of the present invention, the absorbent article is a sanitary napkin of the type disclosed in U.S. Patent No. 3,672,371. It includes an absorbent pad having an elongated main body with a body facing surface and an opposite garment facing surface. A portion of the garment facing surface is coated with a discrete pattern of the hot melt positioning adhesive of the present invention for releasably attaching or locating the absorbent pad in a predetermined position on an article of clothing such as an undergarment which is manufactured of a fibrous substrate such as cotton, nylon, or the like. The absorbent pad typically is enclosed in a fluid-pervious wrapper which can be bound to the pad by the positioning adhesive although other methods of packaging the absorbent pads may be used. Further, a release liner usually covers or overlays the positioning adhesive coating until the pad is employed, at which time the release liner is peeled away thereby exposing the positioning adhesive, and then subsequently discarded. The garment facing portion of the absorbent pad is then pressed against the body facing surface of the undergarment where it is held in place by the positioning adhesive to achieve the benefits described earlier.

In the preferred embodiment of the present invention, the positioning adhesive contains about 15 to 50 parts by weight of an SIS block copolymer including about 25 parts to about 50 parts of styrene per 100 parts of the copolymer; about 30 to about 70 parts by weight of a compatible styrenated terpene tackifying resin; about 5 to about 35 parts of a paraffinic/naphthenic oil and an effective amount of a hindered phenolic antioxidant.

The SIS block copolymer employed in the positioning adhesive may be one of two specific classes. The first class includes unvulcanized elastomeric triblocks or multiblock copolymers wherein the respective monomeric moieties are arranged in an alternating sequence having the general configuration S-I-S and S-I-S-I-S-I, and wherein the styrene provides the nonelastomeric block and the isoprene the elastomeric polymer block. As noted above, the styrene component of the block copolymer ranges from about 25 to about 50 parts per 100 parts copolymer. Suitable styrene-isoprene block copolymers for use herein are available commercially from Enichem Americas under the trade name "Sol T". A preferred block copolymer for use in the compositions of the present invention is Sol T 193B.

The second class of SIS block copolymers which is suitable for the positioning adhesive of the present invention includes a teleblock copolymer having a structure with at least three branches which radially extend outwardly from a central hub. Each of these branches has a polystyrene terminal block and an isoprene segment in the center thereof. This class of SIS block copolymer may also be described, in the alternative, as having a branched polymerized isoprene midblock with a polystyrene terminal block at the end of each branch. Similarly in this instance, the styrene of the SIS block copolymer ranges from about 25 to 50 parts per 100 parts of the block copolymer.

It should be readily recognized that mixtures of the above block copolymers also may be used.

It should be understood, however, that adjustment of the range of styrene content below the lower limit of 25 parts per 100 parts of the SIS block copolymer will produce undesirable results. In this regard, several references, and more particularly the patents to Bunnelle et al., U.S. Patent No. 4,411,954, and St. Clair, U.S. Patent No. 4,835,200 have disclosed the use of SIS block copolymers having styrene compositions below 25 parts per 100 parts of the SIS block copolymer, and which are employed in adhesive applications which are unrelated to the present problem, that is, the placement of an absorbent article on an underlying fibrous substrate; and the release of an absorbent article from a fibrous substrate without depositing residue attributable to the placement adhesives on the fibrous substrate or without disturbing the underlying fibers of the fibrous substrate.

In particular, the reference to St. Clair teaches the use of an SIS block copolymer having less than 25% styrene as being valuable in formulating a construction adhesive which must not release under normal conditions. Further, the reference to Butch III et al., while disclosing an adhesive composition which must release from an underlying substrate, actually teaches away from the composition of the present invention because it clearly teaches that SIS block copolymers should have less than 25 parts styrene per 100 parts of the block copolymer. Finally, and while the Bunnelle et al reference suggests the use of a SIS copolymer having a styrene content of greater than 20% for use as a component in an adhesive composition which is employed for elastic bonding, and which must not release under normal conditions of use, the reference does not teach or suggest the use of an SIS block copolymers with a styrene content greater than 25% and which may be employed as a releasable positioning adhesive for an absorbent article. A further discussion and quanitative comparison of the prior art teachings with that of the present invention will be discussed in greater detail hereinafter.

The compatible tackifying resins which are used in the novel positioning adhesives of the present invention are those which extend the adhesive properties thereof and improve the specific adhesion of the SIS block copolymer. As used herein, the term "tackifying resin" includes, natural and modified rosin such as, for example, gum rosin, wood rosin, tall-oil rosin, distilled rosin, hydrogenated rosin, dimerized rosin and polymerized rosin; glycerol and pentaerythritol esters of natural and modified rosins, such as, for example, the glycerol ester of pale wood rosin, the glycerol ester of hydrogenated rosin, the glycerol ester of polymerized rosin, the pentaerythritol ester of pale wood rosin, the pentaerythritol ester of hydrogenated rosin, the pentaerythritol ester of tall oil rosin and the phenolic modified pentaerythritol ester of rosin; polyterpene resins generally resulting from the polymerization of terpene hydrocarbons, such as the monoterpene known as pinene, in the presence of Friedel-Crafts catalysts at moderately low temperatures; also included are the hydrogenated polyterpene resins; copolymers and terpolymers of natural terpenes, that is, styrene/terpene, alpha-methyl styrene/terpene and vinyl toluene/terpene; phenolic-modified terpene resins such as, for example, the resin product resulting from the condensation, in an acidic medium, of a terpene and a phenol; aliphatic petroleum hydrocarbon resins resulting from the polymerization of monomers consisting primarily of olefins and diolefins; also included are the hydrogenated aliphatic petroleum hydrocarbon resins; aromatic petroleum hydrocarbons and the hydrogenated derivatives thereof. These resins also may improve the elevated temperature resistance of the compound. These resins are materials which tend to associate with the styrene portion of the polymer; aliphatic/aromatic petroleum derived and the hydrogenated derivatives thereof.

It should be understood that mixtures of two or more of the above described tackifying resins may be required for some formulations. Especially preferred in this regard are styrenated terpenes. These styrenated terpenes are commercially available under the trade name Zonatac Lite and which are sold by the Arizona Chemical Co.

Assorted plasticizing oils or extending oils may be used in the novel positioning adhesive of the present invention and include not only the conventional plasticizing oils, familiar to those who are skilled in the art, but also may include olefin oligomers and low molecular weight polymers as well as vegetable and animal oil and derivatives of such oils. The above-identified oligomers include polypropylenes, polybutenes, hydrogenated polyisoprene, hydrogenated butadiene, or the like and which have average molecular weights which lie in a range between about 350 and about 10,000. The aforementioned vegetable and animal oils include glyceryl esters of the usual fatty acids and polymerization products thereof. In this regard, and especially preferred as plasticizing oils are paraffinic and naphthenic hydrocarbons. These petroleum derived process oils have relatively high boiling temperatures and are thus very low in relative volatility and otherwise contain only a minor proportion of aromatic hydrocarbons, that is, less than 30% and more particularly less than 15% by weight of the oil. Alternately, the plasticizing oil may be non-aromatic.

A petroleum derived wax also can be used in the composition of the novel positioning adhesives of the present invention. In this instance, the petroleum derived wax reduces the melt viscosity of the positioning adhesives without appreciably decreasing their adhesive bonding characteristics. Among the applicable

EP 0 525 251 A1

petroleum derived wax additives which may be used to reduce the melt viscosity of the positioning adhesives are low molecular weight, that is, 1000-6000, polyethylene having a hardness value, as determined by ASTM method D-1321, of from about 0.1 to 120 and an ASTM softening point of from about 150 to 250°F; petroleum waxes such as paraffin wax having a melting point of from about 130 to 175°F and Microcrystalline wax having a melting point of from about 135 to 200°F, the latter melting points being determined by ASTM method D127-60; atactic polypropylene having a Ring and Ball softening point of from about 120 to 160°C; and synthetic waxes made by polymerizing carbon monoxide and hydrogen such as Fischer-Tropsch wax. As should be understood, each of these petroleum wax diluents is solid at room temperature. Further, hydrogenated animal, fish and vegetable fats and oils such as hydrogenated tallow, lard, soya oil, cottonseed oil, castor oil, menhadin oil, cod liver oil, etc., are solid at ambient temperature as a result of their being hydrogenated and have also been found useful as a wax diluent equivalent. The aforementioned hydrogenated materials are often referred to in the adhesives industry as "animal or vegetable waxes." Additionally, these wax materials may be incorporated into the positioning adhesives of the present invention in the form of a coating thereby improving the handling characteristics of the positioning adhesives.

Stabilizers which can be incorporated into the positioning adhesives formulation of the present invention are provided to assist in protecting the otherwise vulnerable block copolymer from thermal and oxidative degradation which is frequently encountered during the manufacture and application of the positioning adhesive, as well as in the ordinary exposure of the final end product to ambient environmental conditions. Such degradation is usually characterized by a deterioration in the appearance, physical properties and performance of the positioning adhesive. For example, such degradation may be manifested by a change in color, or alternatively, the positioning adhesive may display a characteristic inability to adhere to the adjacent fibrous substrate or otherwise perform in a fashion which would be unsuitable for the end use application.

The applicable stabilizers include high molecular weight hindered phenols and multifunctional phenols, such as sulfur and phosphorous containing phenols. Hindered phenols are well-known to those skilled in the art and are generally characterized as phenolic compounds which also contain sterically bulky radicals in close proximity to the phenolic hydroxyl group thereof. More particularly, tertiary butyl groups generally are substituted onto the benzene ring in at least one of the ortho positions relative to the phenolic hydroxyl group. It is believed that the presence of these sterically bulky substituted radicals in the vicinity of the hydroxyl group serves to retard its stretching frequency and correspondingly, its reactivity; this steric hindrance thus providing the phenolic compound with its stabilizing properties. Representative hindered phenols include: 1,3,5-trimethyl-2,4,6-tris (3-5-ditert-butyl-4-hydroxybenzyl) benzene; penataerythritol tetrakis-3 (3,5-di-tert-butyl-4-hydroxyphenyl)propionate; n-octadecyl-3 (3,5-di-tert-butyl-4-hydroxyphenyl) propionate; 4,4'-methylenebis (4-methyl-6-tert-butylphenol); 4,4'-thiobis (6-tert-butyl-o-cresol); 2,6-di-tert-butylphenol; 6-(4-hydroxyphenoxy)-2,4-bis (n-octylthio)-1,3,5-triazine; di-n-octadecyl-3,5-di-tert-butyl-4-hydroxybenzylphosphonate; 2-(n-octylthio) ethyl-3,5-di-tert-butyl-4-hydroxybenzoate; and sorbitol hexa-(3,3,5-di-tert-butyl-4-hydroxyphenyl propionate). Especially preferred as a stabilizer is pentaerythritol tetrakis-3 (3,5-di-tert-butyl-4-hydroxyphenyl) propionate.

The performance of these stabilizers may be further enhanced by utilizing, in conjunction therewith synergists such as, for example, thiodipropionate esters and phosphites; and chelating agents and metal deactivators such as, for example, ethylenediaminetetraacetic acid, and salts thereof, and disalicylal-propylenediimine.

The hot melt positioning adhesives of the present invention may be formulated using any of the techniques known in the art. For example, one procedure involves placing all of the plasticizing oil and stabilizers in a jacketed mixing kettle, such as a jacketed heavy duty mixer of the Baker-Perkins or Day type and which is equipped with rotors, and thereafter raising the temperature to a range of from about 250 to 350°F. The precise temperature to be used in the present procedure will depend on the melting point of the particular ingredients. When the initial mixture has been heated the mixture is blanketed in $CO_2$ at a slow flow rate and the resins which were discussed earlier, are slowly added. When the resins are melted, and when the desired temperature is reached, the block copolymer is added to the mixture. The resultant mixture is then agitated until the block copolymer is completely dissolved and a uniform blend is achieved. A vacuum is then applied to the mixing kettle thereby removing any entrapped air which is present. The composition can then be cooled and packaged for later use.

The invention is further illustrated by an example which follows.


EXAMPLE 1

5

A hot melt positioning adhesive in accordance with the present invention was made by the procedure described above. Further, this positioning adhesive has the following composition:

30 parts of an SIS block copolymer having a composition of 25% styrene and which is manufactured under the trademark, Sol T 193 B by the Enichem Americas Company;

50 parts of a styrenated terpene which is manufactured under the trademark Zonatac 501 Lite by The Arizona Chemical Company;

20 parts of a paraffinic/naphthenic oil which is manufactured under the trade name of Kaydol by the Witco Chemical Company; and

0.5 parts of a hindered phenolic antioxidant which is manufactured under the trade name Irganox 1010 by the Ciba-Geigy Company.

The resulting adhesive composition had a viscosity of approximately 5,250 cps at about 325° F.

The resulting hot-melt positioning adhesive, was subsequently heated to a temperature where it flowed readily (330° F), and was extruded thereafter directly onto the garment facing surface of an absorbent pad. Alternatively, it could be reverse (transfer) coated onto release paper employed with same by using any of several techniques known in the art, including flow coating, roller coating, knife coating, or the like. The positioning adhesive also could be sprayed into place by using a hot-melt sprayer.

The positioning adhesive composition which was formulated in the manner noted above was evaluated using the test procedures which are set forth below.

Samples of the positioning adhesive were prepared for testing by coating an approximate 2 mil thickness of the positioning adhesive on a 2 mil thick Mylar™ substrate. Mylar is a trademark of The Du Pont Chemical Corporation. After conditioning overnight, sample strips (1 inch by 5 inches) are cut out.

Initial Cotton Peel:

The coated samples are pressed into adhesive contact with a cotton fabric substrate (on a flat surface) by using two passes with a 4.5 lb. (2 kg) rubber roller. After the application of force, the coated samples are pulled away from the cotton fabric in a peel mode by employing an Instron Tensile Tester at a crosshead speed of 20 inches (50 cm) per minute. An average peel adhesion of at least five samples is recorded in grams.

Use Cotton Peel:

The coated samples are further pressed into contact with a cotton fabric substrate with only light finger pressure. Following the application of force, the coated samples are placed on a small flat surface and a load of approximately 125 grams per square inch is applied to same. The flat surface is then placed in an oven at approximately 105° F (40° C) for a period of approximately 4 hours. Following this time interval, the coated samples are removed from the oven and allowed to equilibrate to the ambient room temperature.

The coated samples are then separated from the cotton fabric substrate in a peel mode by employing an Instron Tensile Tester at a crosshead speed of approximately 20 inches (50 cm) per minute. An average peel adhesion of at least five samples is recorded in grams.

Transfer:

Coated samples, as described above, are pressed into contact with a cotton fabric substrate with only light finger pressure. The coated samples are then placed on a small flat surface and a load of approximately 1250 grams per square inch is applied to same. The flat surface is then placed in an oven at approximately 105° F (40° C) for a period of approximately 16 hours. Following this time period, the samples are removed from the oven and allowed to equilibrate to ambient room temperature. The coated samples are then separated from the cotton fabric substrate in a peel mode by employing an Instron Tensile Tester at a crosshead speed of approximately 20 inches (50 cm) per minute. The average peel adhesion of at least three samples is recorded in grams. After the peel adhesive test is performed, the amount of adhesive residue deposited on the cotton fabric is noted. Transfer testing can also be repeated with nylon fabric. The results of these tests are also provided hereinafter.

Heat Stability:

In this test, two hundred gram samples of molten positioning adhesive, which was formulated using the method described earlier, are poured into clean glass jars and covered with aluminum foil. The adhesive samples are then placed in a forced air recirculatory oven maintained at a temperature of approximately 350°F ± 5°F. The adhesive samples are then evaluated after time intervals of 8, 24, and 48 hours for changes in color, phase separation and gelation. Changes in the viscosity of these samples are measured and compared against the initial viscosity.

The results of the tests, above, indicate that the composition of Example 1 is sufficiently aggressive and possesses adequate tack to bind an absorbent pad securely to fabrics which have been traditionally used to manufacture undergarments. The positioning composition also was found to cleanly release from these fabrics without leaving any undesirable adhesive residues.

Examples 2 through 10

In the following examples, hot melt positioning adhesives in accordance with the present invention were manufactured using the procedure described earlier. Further, these same positioning adhesives had compositions as set forth in the following table.

| EXAMPLES | 2 | 3 | 4 | 5 |
|---|---|---|---|---|
| Ingredients (in parts) | | | | |
| Kaydol | 20 | 20 | 20 | 20 |
| Zonatac 501 Lite | 50 | 50 | 50 | 50 |
| Kraton D 1107(a) | 30 | | | |
| Kraton D 1112(a) | | 30 | | |
| Kraton D 1117(a) | | | 30 | |
| Kraton D 1111(a) | | | | 30 |
| Sol T - 193B(b) | | | | |
| Dexco DPX 506(c) | | | | |
| Kraton RP - 6405(a) | | | | |
| Dexco DPX 5276-29(c) | | | | |
| Dexco DPX 505(c) | | | | |
| Irganox 1010 | 1 | 1 | 1 | 1 |
| % Styrene | 14% | 14% | 17% | 21% |
| Viscosity (cP. @ 325°F) | 12,700 | 10,770 | 4,380 | 13,350 |
| Initial Cotton Peel (Grams) | 429 | 511 | 475 | 357 |
| Use Cotton Peel (Grams) | 427 | 515 | 585 | 242 |
| Transfer | | | | |
| Cotton Peel (Grams) | 2,256 | 2,201 | 1,765 | 495 |
| Qualitative Residue | Moderate | Heavy | Moderate | None |
| Nylon Peel (Grams) | 2,103 | 1,990 | 2,145 | 970 |
| Qualitative Residue | Moderate | Moderate | Moderate | Slight |

| EXAMPLES | 6 | 7 | 8 | 9 | 10 |
|---|---|---|---|---|---|
| Ingredients (in parts) | | | | | |
| Kaydol | 20 | 20 | 20 | 20 | 20 |
| Zonatac 501 Lite | 50 | 50 | 50 | 50 | 50 |
| Kraton D 1107(a) | | | | | |
| Kraton D 1112(a) | | | | | |
| Kraton D 1117(a) | | | | | |
| Kraton D 1111(a) | | | | | |
| Sol T - 193B(b) | 30 | | | | |
| Dexco DPX 506(c) | | 30 | | | |
| Kraton RP - 6405(a) | | | 30 | | |
| Dexco DPX 5276-29(c) | | | | 30 | |
| Dexco DPX 505(c) | | | | | 30 |
| Irganox 1010 | 1 | 1 | 1 | 1 | 1 |
| % Styrene | 25% | 28% | 30% | 32% | 42% |
| Viscosity (cP. @ 325°F) | 5,625 | 4,220 | 3,875 | 2,625 | 2,890 |
| Initial Cotton Peel (Grams) | 403 | 311 | 330 | 285 | 219 |
| Use Cotton Peel (Grams) | 260 | 216 | 228 | 253 | 135 |
| Transfer | | | | | |
| Cotton Peel (Grams) | 1,075 | 881 | 821 | 808 | 630 |
| Qualitative Residue | Slight | None | None | None | None |
| Nylon Peel (Grams) | 1,352 | 1,136 | 1,063 | 941 | 695 |
| Qualitative Residue | Slight | Slight | None | Slight | None |

(a) An SIS Block Copolymer which is manufactured by The Shell Chemical Company.

(b) An SIS Block Copolymer which is manufactured by The Enichem Chemical Company.

(c) An SIS Block Copolymer which is manufactured by The Dexco Chemical Company.

An analysis was conducted of the relationship of viscosity as compared with the percent styrene concentration in the particular SIS block copolymers being analyzed. More particularly, it will be noted following a study of the information, above, that those samples manufactured with an SIS block copolymer having a styrene concentration of greater than 25% had a much more favorable viscosity as measured in centipoise (cP) at 325°F. This was surprising in light of the viscosity measurements of placement adhesive compositions having SIS block copolymers with percentage styrene concentrations of less than 25%. In particular, it should be noted that the viscosity of the individual examples appear to remain constant or increase when the percentage of styrene is increased from 14% to 21%. Moreover, the tests employed to determine the suitability of the placement adhesive, vis-a-vis the deposit of adhesive residue on both cotton and nylon substrates indicates that increasing the styrene concentration does not appear to have any substantial effect with regards to reducing the undesirable characteristics of the placement adhesive to deposit adhesive residue on the cloth substrates used in the present tests. In light of the foregoing, it was unexpected that increasing the concentration of styrene in the SIS block copolymers in excess of 25% would result in unusually desireable viscosity characteristics, and would additionally permit the positioning adhesive to release easily from the fabric substrates, such as cotton or nylon without depositing substantial amounts of adhesive residue.

Examples 10 through 35

In the following examples, various hot melt positioning adhesives manufactured in accordance with the prior art teachings of Collins et al., Patent No. 4,136,699; Butch III, Patent No. 4,411,954; Raykovitz, Patent No. 4,704,110; Sieverding, Patent No. 4,835,200, which are the closest prior art that the inventors are aware of, were compared and contrasted with additional examples of positioning adhesives made in accordance with teachings of the present invention to demonstrate the superior performance and unexpected results achieved by the present adhesive formulation.

In the following examples, which are displayed in graphic format, the following prior art teachings relate to the individual examples:

Example 11      A sample of placement adhesive which has physical characteristics that are outside the lowermost range of the present invention;

Examples 12, 13, 14      Samples of placement adhesive made in accordance with the present invention, and wherein the styrene content has been varied within the range specified to demonstrate the advantageous viscosities achieved;

Example 15      An adhesive composition made in accordance with the teachings of Collins et al., U.S. Patent No. 4,136,699;

Examples 16-19      An adhesive composition made in accordance with the teachings of Butch III, U.S. Patent No. 4,411,954;

Examples 20-26      An adhesive composition made in accordance with the teachings of Raykovitz, U.S. Patent No. 4,704,110;

Example 27      An adhesive composition made in accordance with the teachings of Sieverding, U.S. Patent No. 4,833,193;

Example 28      An adhesive composition made in accordance with the teachings of Lakshmanan, U.S. Patent No. 4,857,594; and

Examples 29-35      An adhesive composition made in accordance with the teachings of St. Clair, U.S. Patent No. 4,835,200;

EP 0 525 251 A1

| EXAMPLES | 11 | 12 | 13 | 14 | 15 |
|---|---|---|---|---|---|
| Kaydol | 20 | 20 | 20 | 20 | |
| Shellflex 371 | | | | | 30.57 |
| Zonatac 501L | 50 | 50 | 50 | 50 | |
| Wingtac 95 | | | | | 54.0 |
| Kraton D 1112 | 30 | | | | |
| Solt 193B | | 30 | | | |
| Dexco DPX 505 | | | 30 | | |
| Kraton RP 6405 | | | | 30 | |
| Kraton G 1650 | | | | | 15 |
| Irganox 1010 | 1 | 1 | 1 | 1 | 0.5 |
| Kraton G 1657 (SEBS 13% Styrene) | | | | | |
| Kraton D 1107 (SIS 14% Styrene) | | | | | |
| % Styrene | 14 | 25 | 42 | 30 | 29 |
| Vicosity (cP @ 325°F) | 7625 | 5719 | 2738 | 4375 | 2850 |
| Polymer type | SIS | SIS | SIS | SIS | SEBS |
| Inital Cotton Peel (grams) | 508 | 391 | 182 | 339 | 246 |
| Use Cotton Peel (grams) | 475 | 208 | 102 | 173 | 164 |
| Transfer | | | | | |
| Cotton Peel (grams) | 1790 | 822 | 498 | 656 | 519 |
| Qualitative Residue | Heavy | Slight | None | None | None |
| Nylon Peel (grams) | 1727 | 1063 | 668 | 721 | 697 |
| Qualitative Residue | Heavy | Slight | None | None | None |

10

| EXAMPLES | 16 | 17 | 18 | 19 |
|---|---|---|---|---|
| Kaydol | 20 | 20 | 21.5 | 17.5 |
| Shellflex 371 | | | | |
| Zonatac 501L | | | | |
| Wingtac 95 | 55 | 55 | 58.5 | 52.5 |
| Kraton D 1112 | | | | |
| Solt 193B | | | | |
| Dexco DPX 505 | | | | |
| Kraton RP 6405 | | | | |
| Kraton G 1650 | | | | |
| Irganox 1010 | 0.5 | 0.5 | 0.5 | 0.5 |
| Kraton G 1657 (SEBS 13% Styrene) | 20 | 15 | 14 | 21 |
| Kraton D 1107 (SIS 14% Styrene) | 5 | 10 | 6 | 9 |
| % Styrene | | | | |
| Vicosity (cP @ 325°F) | 10125 | 10063 | 4500 | 20500 |
| Polymer type | BLEND | BLEND | BLEND | BLEND |
| Inital Cotton Peel (grams) | 346 | 395 | 471 | 291 |
| Use Cotton Peel (grams) | 254 | 253 | 387 | 172 |
| Transfer | | | | |
| Cotton Peel (grams) | 1097 | 992 | 1259 | 774 |
| Qualitative Residue | Moderate | Slight | Moderate | None |
| Nylon Peel (grams) | 1282 | 1198 | 1509 | 987 |
| Qualitative Residue | Moderate | Moderate | Moderate | Slight |

| EXAMPLES | 20 | 21 | 22 | 23 | 24 |
|---|---|---|---|---|---|
| Stereon 845 | 27.5 | 27.5 | 27.5 | 27.5 | 27.5 |
| Kraton 1102 | | | | | |
| Stereon 840A | | | | | |
| ArcoPrime 400 | | | | | |
| Vestoplast 508 | | | | | |
| Shellflex 371 | 7.5 | 7.5 | 7.5 | 7.5 | 7.5 |
| Kraton G 1657 | | | | | |
| Kraton G 1651 | | | | | |
| Kraton G 1701 | | | | | |
| Wingtack 10 | 10 | 10 | 10 | 10 | 10 |
| Arkon M 100 | 55 | | | | |
| Zonatac 105L | | 55 | | | |
| Escorez 5300 | | | 55 | | |
| Foral 105 | | | | 55 | |
| Wingtack 86 | | | | | 55 |
| Zonatac 501L | | | | | |
| Regalrez 1018 | | | | | |
| Escorez 5380 | | | | | |
| Irganox 1010 | 0.25 | 0.25 | 0.25 | 0.25 | 0.25 |
| Irgaphos 168 | 0.25 | 0.25 | 0.25 | 0.25 | 0.25 |
| % Styrene | 48 | 48 | 48 | 48 | 48 |
| Polymer type | SBS | SBS | SBS | SBS | SBS |
| Viscosity (eP @ 325°F) | 6500 | 8475 | 9500 | 8800 | 7175 |
| Inital Cotton Peel (grams) | 128 | 55 | 0 | 86 | Not Performed |
| Use Cotton Peel (grams) | 127 | 170 | 55 | 111 | -- |
| Transfer | | | | | |
| Cotton Peel (grams) | 890 | 1002 | 306 | 835 | -- |
| Qualitative Residue | None | None | None | None | -- |
| Nylon Peel (grams) | 989 | 808 | 98 | 1130 | -- |
| Qualitative Residue | None | None | None | Slight | -- |

| EXAMPLES | 25 | 26 | 27 | 28 |
|---|---|---|---|---|
| Stereon 845 | | | | |
| Kraton 1102 | 27.5 | | | |
| Stereon 840A | | 27.5 | | |
| ArcoPrime 400 | | | 60 | |
| Vestoplast 508 | | | | 47.5 |
| Shellflex 371 | 7.5 | 7.5 | | |
| Kraton G 1657 | | | | 5 |
| Kraton G 1651 | | | 3 | |
| Kraton G 1701 | | | 4 | |
| Wingtack 10 | 10 | 10 | | |
| Arkon M 100 | | | | |
| Zonatac 105L | | | | |
| Escorez 5300 | | | | |
| Foral 105 | | | | |
| Wingtack 86 | | | | |
| Zonatac 501L | 55 | 55 | | |
| Regalrez 1018 | | | 33 | |
| Escorez 5380 | | | | 47.5 |
| Irganox 101 | 0.25 | 0.25 | | 0.5 |
| Irgaphos 168 | 0.25 | 0.25 | | |
| % Styrene | 28 | 43 | | 13 |
| Polymertype | SBS | SBS | Blend | Blend |
| Viscosity (cP @ 325°F) | 11200 | 7175 | 463 | 7500 |
| Inital Cotton Peel (grams) | 303 | 136 | 50 | 19 |
| Use Cotton Peel (grams) | 304 | 236 | 16 | 100 |
| **Transfer** | | | | |
| Cotton Peel (grams) | 1792 | 1711 | 43 | 641 |
| Qualitative Residue | Moderate | Slight | None | None |
| Nylon Peel (grams) | 1997 | 1588 | 57 | 169 |
| Qualitative Residue | Heavy | Slight | None | None |

| EXAMPLES | 29 | 30 | 31 | 32 | 33 |
|---|---|---|---|---|---|
| Kraton D 1102 | 25 | | | | |
| Kraton D 1107 | | 25 | | | |
| Kraton D1320 | | | 25 | | |
| Kraton G1657 | | | | 25 | |
| Stereon 840A(a) | | | | | 25 |
| Escorez 5300 | 60 | 60 | 60 | 60 | 60 |
| Drakeol Supreme (b) | 15 | 15 | 15 | 15 | 15 |
| Irganox 1010 | 0.5 | 0.5 | 0.5 | 0.5 | 0.5 |
| % Styrene | 28 | 14 | 10 | 13 | 43 |
| Viscosity (cP @ 325°F) | 8000 | 11750 | 20500 | 18000 | 6719 |
| Polymer Type | SBS | SIS | (SI)n | SEBS | SBS |
| Inital Cotton Peel | 14 | 358 | 194 | 189 | 0 |
| Use Cotton Peel | 142 | 244 | 94 | 278 | 58 |
| Transfer | | | | | |
| Cotton Peel (grams) | 1020 | 1285 | 575 | 1402 | 471 |
| Qualitative Residue | Moderate | Slight | None | Moderate | None |
| Nylon Peel (grams) | 902 | 1508 | 1097 | 1947 | 287 |
| Qualitative Residue | Slight | Slight | Slight | Heavy | None |

| EXAMPLES | 34 | 35 |
|---|---|---|
| Kraton D 1102 | | |
| Kraton D 1107 | 20 | |
| Kraton D1320 | | |
| Kraton G1657 | | |
| Stereon 840A(a) | | 20 |
| Escorez 5300 | 60 | 60 |
| Drakeol Supreme (b) | 20 | 20 |
| Irganox 1010 | 0.5 | 0.5 |
| % Styrene | 14 | 43 |
| Viscosity (cP @ 325°F) | 3906 | 2750 |
| Polymer Type | SIS | SBS |
| Inital Cotton Peel | 408 | 73 |
| Use Cotton Peel | 397 | 195 |
| | | |
| Transfer | | |
| | | |
| Cotton Peel (grams) | 1768 | 810 |
| | | |
| Qualitative Residue | Moderate | None |
| | | |
| Nylon Peel (grams) | 1596 | 692 |
| | | |
| Qualitative Residue | Moderate | Slight |

(a)   manufactured by the Firestone Chemical Company
(b)   manufactured by the Penreco Company

After a thorough analysis of the data presented above, it should be readily recognized that the prior art teachings cannot achieve the benefits of the present invention vis-a-vis an advantageous viscosity, and the ability of the placement adhesive of the present invention to release easily, and cleanly, from an adjoining fabric substrate without depositing an adhesive residue on the substrate.

In particular, it should be noted that with respect to example 11, an adhesive composition was formulated which employs a copolymer having a styrene content outside the lowermost range of the present invention. When tested, using the earlier described methods, it will be noted that the adhesive deposits an unacceptable amount of adhesive residue on the underlying fabric substrate, and further has an unacceptably high viscosity of 7625. With respect to examples 12, 13, and 14 it will be seen that these adhesive samples were made in accordance with the present invention, however, in each instance, the Styrene content was varied to demonstrate the characteristics of adhesive. It will be noted that each sample released cleanly from the underlying fabric substrate and that acceptable viscosities of 4910; 2738; and 4375 cP, respectively were achieved. Example 15 was provided to compare the teachings of the prior art patent to Collins, U.S. Patent No. 4,136,699 with the present invention. In this regard, it will be seen that while the adhesive material had satisfactory release properties and viscosity, as disclosed in Raykovitz 4,704,110, the composition required "the use of specific block copolymers wherein the mid-block is hydrogenated and wherein relatively large amounts of oil are needed in proportion to the amount of block copolymer. These hydrogenated copolymers are relatively expensive raw materials and are difficult to tackify." Examples 16-19 were formulated in accordance with the teachings of Butch III et al., U.S. Patent No. 4,411,954. A review of this information reveals that these adhesive formulations were unsuitable inasmuch as they deposited an undesirable amount of adhesive residue and/or had undesirable viscosities of 10,125; 10,063; 4,500; and 20,500, respectively. Examples 20-26 relate to the teachings found in the Raykovitz Patent No. 4,704,110. It will be seen following a study of these references and the data, above, that each of the adhesive compositions were undesirable inasmuch as the viscosities were unnecessarily high, that is 6500; 8475; 7500; 8800; 7175; 11200 and 7175, respectively. Examples 27-35 had similar undesirable characteristics. It should be apparent, therefore, that the prior art does not provide for an adhesive composition having the characteristics displayed by the present adhesive positioning formulation.

Operation

The operation of the described embodiment of the present invention is believed to be readily apparent and is briefly summarized at this point.

The positioning adhesive of the present invention finds great utility when used in combination with an absorbent article which is adhesively attached to a fibrous substrate. In this regard, the placement adhesive permits the absorbent article to be releasably attached to the fabric substrate without depositing a substantial amount of residue attributable to the adhesive on the substrate. Further, the positioning adhesive of the present invention does not substantially rend or otherwise disturb the fiber composition of the substrate. In the present invention, the absorbent pad has a main body with opposite first and second surfaces, and the first surface is adapted to be placed in contact with the substrate which may be an undergarment or the like. The positioning adhesive is then applied in a discreet pattern on the first surface of the absorbent pad for releasably attaching the absorbent pad to the substrate. As discussed earlier, the pressure sensitive positioning adhesive of the present invention has a viscosity of about 2000 to 5000 centipoise at a pour temperature of approximately 325°F and an initial cotton peel adhesion of about 200 to about 400 grams. The positioning adhesive further has a composition including about 30 parts of a styrene-isoprene-styrene block copolymer containing at least 25 parts styrene per 100 parts of the copolymer; about 50 parts of a terpene tackifying resin; about 20 parts of a plasticizing oil; and an effective amount of stabilizer. Alternatively, the pressure sensitive positioning adhesive may consist essentially of about 15 to about 50% by weight of a styrene-isoprene - styrene block copolymer containing from about 25 parts to about 50 parts styrene per 100 parts of the copolymer; about 30% to about 70% by weight of a compatible tackifying resin; and about 5% to about 35%, by weight of a plasticizing oil.

Therefore, it will be seen that the placement adhesive of the present invention provides a fully dependable and practical means for adhesively attaching an absorbent article, such as a feminine napkin, or the like, on an article of clothing which includes a fabric substrate, and further provides a means whereby the absorbent article may be conveniently released from the fabric substrate without depositing any substantial adhesive residue on the fabric substrate. In addition to the foregoing, the placement adhesive, which is used in combination with the absorbent article, provides a composition which is characterized by a viscosity which lies in a range of about 2000 to 5000 centipoise, at a pour temperature of 325°F, and initial cotton peel adhesion of about 200 to about 400 grams.

It will be apparent to those skilled in the art that the foregoing examples have been made for the purposes of illustration and that variations may be made in proportions, procedures and materials without departing from the scope of the present invention. Therefore, it is intended that this invention not be limited except by the claims which follows:

**Claims**

1. An absorbent article which is adhesively attached to a fibrous substrate and wherein the absorbent article can be easily released from the substrate without depositing a substantial amount of residue attributable to the adhesive on the substrate, and which further does not disturb the fibers of the substrate, the absorbent article comprising:

an absorbent pad having a main body with opposite first and second surfaces, and wherein the first surface is operable to be placed in contact with the substrate,

a pressure sensitive positioning adhesive applied in a discrete pattern on the first surface of the absorbent pad for releasably attaching the absorbent pad on the substrate, the pressure sensitive positioning adhesive having a viscosity of about 2000 to 5000 centipoise at a pour temperature of approximately 325°F and an initial cotton peel adhesion of about 200 to 400 grams, the pressure sensitive positioning adhesive further comprising,

(a) about 30 parts of a styrene-isoprene-styrene block copolymer containing at least about 25 parts styrene per 100 parts of the copolymer;

(b) about 50 parts of a tackifying resin;

(c) about 20 parts of a plasticizing oil; and

(d) an effective amount of stabilizer.

2. An absorbent article as claimed in claim 1 and wherein the tackifying resin is a single resin or a mixture thereof selected from the group consisting of:

(a) rosins and modified rosins including hydrogenated versions;

(b) glycerol and pentaerythritol esters of rosins and modified rosins including hydrogenated versions;

(c) polyterpene resins;

(d) copolymers and terpolymers of natural terpenes;

(e) phenolic modified terpenes;

(f) aliphatic petroleum hydrocarbon resins including hydrogenated versions;

(g) aromatic petroleum hydrocarbon resins including hydrogenated versions; and

(h) aliphatic/aromatic hydrocarbon resins including hydrogenated versions thereof.

3. An absorbent article as claimed in claim 1 and wherein the tackifying resin is a styrenated terpene.

4. An absorbent article as claimed in claim 1 and wherein the plasticizing oil is a paraffinic or naphthenic oil.

5. An absorbent article as claimed in claim 1 and wherein the placement adhesive further includes about 0.1% to about 2% by weight of an antioxidant/stabilizer.

6. An absorbent article as claimed in claim 1 and wherein the placement adhesive further includes up to about 5% by weight of a wax.

7. A releasable absorbent article for a garment including an absorbent pad having a first or garment facing surface, and wherein a portion of the garment facing surface is coated with a hot melt pressure sensitive adhesive which releases from the garment without substantially depositing any adhesive residue on the garment, the pressure sensitive adhesive having a viscosity of about 2000 to about 5500 cP at a pour temperature of 325°F, the hot melt pressure sensitive adhesive consisting essentially of:

about 15 to about 50% by weight of a styrene-isoprene-styrene block copolymer containing from about 25 parts to about 50 parts styrene per 100 parts of the copolymer;

about 30% to about 70%, by weight, of a compatible tackifying resin; and

about 5% to about 35% by weight of a plasticizing oil.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-9 102 039 (THE DOW CHEMICAL COMPANY) * page 6, line 17 - page 10, column 14; claims; examples * | 1-7 | A61L15/58 C09J153/00 |
| X,D | US-A-4 835 200 (D. ST.CLAIR) * claims; examples * | 1-7 | |
| X | US-A-5 028 646 (J. MILLER) * column 4, line 60; claims; examples * | 1-7 | |
| X | EP-A-0 101 961 (NATIONAL STARCH AND CHEMICAL CORPORATION) * claims; examples * | 1-7 | |
| D | & US-A-4 411 954 | | |
| P,X | EP-A-0 451 919 (SHELL INTERNATIONALE RESEARCH MAATSCHAPPIJ) * the whole document * | 1-7 | |
| A | EP-A-0 410 412 (H.B.FULLER COMPANY) * page 12, line 29 - line 50; claims; examples * | 1-7 | TECHNICAL FIELDS SEARCHED (Int. Cl.5) |
| A | US-A-4 526 577 (R. SCHMIDT) | | A61L C09J |

----

The present search report has been drawn up for all claims

| Place of search | Date of completion of the search | Examiner |
|---|---|---|
| THE HAGUE | 13 OCTOBER 1992 | G.COUSINS-VAN STEEN |